# FASCICULE DE BREVET EUROPEEN

(11) **EP 1 066 822 B1**
(45) Date de publication et mention de la délivrance du brevet: **06.10.2004**
(21) Numéro de dépôt: 00401549.1
(22) Date de dépôt: 31.05.2000
(51) Int. Cl.: A61K 7/075, A61K 7/06

(54) **Composition de traitement antipelliculaire des cheveux et du cuir chevelu, à base d'actif antipelliculaire et d'hydroxyacide**
Zusammensetzung zur Behandlung der Haarschuppen auf dem Haar oder der Kopfhaut basiert auf einem Antischuppenmittel und einer Hydroxysäure
Composition for treating dandruffs in hair and scalp based on an antidandruff agent and a hydroxy-acid

(30) Priorité: 08.07.1999 FR 9908877
(43) Date de publication de la demande: 10.01.2001
(60) Demande divisionnaire: 04290757.6
(73) Titulaire: L'OREAL, 75008 Paris (FR)
(72) Inventeur: Maurin, Véronique, 75016 Paris (FR); Beauquey, Bernard, 92110 Clichy (FR)
(74) Mandataire: Dodin, Catherine

(56) Documents cités:
- EP-A- 0 273 202
- WO-A-98/55093
- GB-A- 2 180 846
- DATABASE CHEMICAL ABSTRACTS [en ligne] STN; abrégé 121: 308 342, XP002131948 & JP 06 234618 A (KAO CORP.) 23 août 1994 (1994-08-23)

## Description

La présente invention concerne des compositions cosmétiques de lavage et de traitement antipelliculaire des cheveux et du cuir chevelu, à base d'agent antipelliculaire, d'hydroxyacide et d'agent tensioactif non-ionique du type alkyl(poly)glycoside.

En vue de combattre la formation des pellicules qui est généralement accompagnée d'une prolifération microbienne et/ou fongique, il a été proposé comme produits anti-pelliculaires soit des produits inhibant la prolifération microbienne, soit des produits kératolytiques. Parmi ces agents antipelliculaires, l'emploi des sels de pyrithione a été tout particulièrement préconisé. De nombreux brevets décrivent les sels de pyridinethione dans les shampooings , par exemple US 3753916, US4345080, US5037818. Les tensioactifs utilisés dans les shampooings sont le plus souvent les tensioactifs anioniques.

WO 9855093 décrit un shampooing contenant un sel de pyridinethione, du lauroamphoacétate de sodium et 2% d'acide malique.

GB 2 180 846 décrit un shampooing contenant un sel de pyridithione, un tensioactif anionique et 1% d'acide citrique.

Il a également été proposé, dans EP-A-0422508 l'utilisation dans des shampooings, d'agent antibactériens tels que des sels de pyridinethione en association avec un agent tensioactif non ionique du type alkylpolyglucoside.

Cependant, la Demanderesse a constaté que ces shampoings présentaient une activité antifongique en particulier vis-à-vis de Malassezia Ovalis encore insuffisante. De plus, les sels de pyridinethione étant insolubles dans l'eau, ils peuvent également poser des problèmes de mise en suspension s'ils sont présents à des taux relativement importants.

On a maintenant constaté de façon surprenante et inattendue qu'il était possible d'obtenir des compositions notamment cosmétiques de lavage et de traitement antipelliculaire des cheveux et du cuir chevelu présentant une efficacité antipelliculaire améliorée en associant au moins un agent antipelliculaire de type pyridinethione, au moins 1% en poids d'un hydroxyacide et au moins un agent tensioactif choisi parmi les tensioactifs non-ioniques du type alkyl(poly)glycoside.

L'invention a donc pour objet une composition de lavage et de traitement antipelliculaire des cheveux et du cuir chevelu, comprenant dans un milieu aqueux au moins un agent antipelliculaire choisi parmi les sels de pyridinethione, au moins 1% en poids d'au moins un hydroxyacide et au moins un agent tensioactif choisi parmi les tensioactifs non-ioniques du type alkylpolyglycoside.

Un autre objet de invention consiste en un procédé de lavage et de traitement cosmétique pour l'élimination des pellicules des cheveux ou du cuir chevelu utilisant ces compositions.

D'autres objets apparaîtront à la lumière de la description et des exemples qui suivent.

Les sels de pyridinethione sont notamment choisis parmi les sels de calcium, magnésium, barium, strontium, zinc, cadmium, étain et de zirconium. Le sel de zinc est particulièrement préféré.

De tels composés sont notamment commercialisés sous la dénomination Omadine de zinc par la société OLIN.

Le ou les sels de pyridinethione sont présents dans les compositions conformes à l'invention dans des proportions comprises de préférence entre 0,1 et 5% en poids et plus particulièrement entre 0,3 et 2,5% en poids par rapport au poids total de la composition.

Selon une caractéristique essentielle des compositions détergentes selon l'invention, ces dernières contiennent au moins 1% en poids d'un hydroxyacide ou de ses dérivés.

Par dérivés d'acide, on entend ses sels associés (sels avec une base organique ou un alcalin notamment) ou bien encore éventuellement son lactide correspondant (forme obtenu par autoestérification des molécules).

Cet hydroxyacide peut bien entendu consister en un acide mono ou poly carboxylique comportant une ou plusieurs fonctions hydroxy, de préférence hydroxyacide est un alpha hydroxy acide : l'une au moins de ces fonctions hydroxy devant occuper une position alpha sur ledit acide (carbone adjacent à une fonction carboxylique). Cet acide peut se présenter dans la composition détergente finale sous forme d'acide libre et/ou sous la forme de l'un de ses sels associés (sels avec une base organique ou un alcalin notamment), en particulier selon le pH final imposé à la composition, ou bien encore éventuellement sous la forme du lactide correspondant (forme obtenu par autoestérification des molécules). Les compositions détergentes conformes à l'invention peuvent bien entendu contenir un plusieurs hydroxyacides ou leurs dérivés.

A titre d'exemples de tels composés, on peut citer, entre autres, les acides citrique, lactique, méthyllactique, phényllactique, malique, mandélique, glycolique, tartronique, tartrique, gluconique, benzylique et l'acide 2-hydroxycaprylique. D'autres composés de type hydroxyacides convenant à la présente invention sont ceux cités dans la demande de brevet EP-A- 0 413 528, dont l'enseignement est, à cet égard, totalement inclus dans la présente demande.
De préférence, on retiendra les acides qui sont cosmétiquement compatibles et acceptables avec les cheveux, la peau et/ou le cuir chevelu.

Selon un mode particulièrement préféré de réalisation de la présente invention, hydroxyacide mis en oeuvre est choisi parmi l'acide citrique, l'acide tartrique et l'acide lactique.

Selon une autre caractéristique importante des compositions détergentes selon l'invention, le ou les hydroxyacides sont présents dans ces dernières à raison d'au moins 1 % en poids, de préférence au moins 2 % en poids par rapport à l'ensemble de la composition. Des teneurs en hydroxyacide(s) comprises entre 2 et 10 % en poids, et plus particulièrement encore entre 2 et 5 % en poids, conviennent généralement très bien. On notera que ces concentrations sont nettement supérieures à celles qui peuvent parfois se rencontrer dans des shampooings de l'art antérieur lorsque certains acides ont été employés uniquement à des fins d'ajustement de pH.

Le ou les agents tensioactifs non ioniques de type alkyl(poly)glycoside, utilisés dans le cadre de la présente invention, sont des produits bien connus en soi, et ils peuvent être plus particulièrement représentés par la formule générale (I) suivante :

R₁-O-(R₂O)ₜ-(G)ᵥ (I)

dans laquelle R₁ représente un radical alkyle linéaire ou ramifié, saturé ou insaturé, linéaire ou ramifié comportant environ de 8 à 24 atomes de carbone, un radical alkylphényle dont le radical alkyle linéaire ou ramifié comporte environ de 8 à 24 atomes de carbone, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.

Des alkyl(poly)glycosides préférés selon la présente invention sont des composés de formule (I) dans laquelle R₁ désigne plus particulièrement un radical alkyle saturé ou insaturé, linéaire ou ramifié comportant de 8 à 14 atomes de carbone, t désigne une valeur allant de 0 à 3 et plus particulièrement encore égale à 0, G désigne le glucose, le fructose ou le galactose, de préférence le glucose. Le degré de polymérisation (S) du saccharide, i.e. la valeur de v dans la formule (I), peut aller de 1 à 15. Selon l'invention, on préfère les sucres réduits contenant 80%, ou plus, de sucres dont le degré de polymérisation (S) prend une valeur allant de 1 à 4. Le degré moyen de polymérisation est plus particulièrement compris entre 1 et 2.

Des composés de formule (I) sont notamment représentés par les produits vendus par la société HENKEL sous la dénomination APG, tels que les produits APG 300, APG 350, APG 500, APG 550, APG 625, APG base 10-12, sous les dénominations PLANTAREN® (1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK. Le(s) agent(s) tensioactif(s) non ioniques sont généralement présents à raison de 0,5 à environ 15% en poids, de préférence de 1 à 5% en poids, par rapport au poids total de la composition.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif est généralement choisi parmi les agents tensioactifs anioniques.

Les tensioactifs additionnels anionique convenant à la mise en oeuvre de la présente invention sont notamment les suivants :
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

Les compositions cosmétiques selon l'invention peuvent se présenter sous forme de liquides plus ou moins épaissis, de gels, de crèmes, de mousses aérosol.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'ethanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à C₁₆, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents hydratants, d'autres agents anti-pelliculaires ou anti-séborrhéiques, des filtres solaires et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou amphotères ou cationiques, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les silicones, volatiles ou non volatiles, solubles et insolubles dans le milieu, les huiles végétales, les huiles de synthèses telles que les poly-alphaoléfines, les huiles fluorées ou perfluorées, et leurs mélanges.

Les compositions selon l'invention comprennent de préférence au moins un polymère cationique et ou une silicone.

Parmi les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les polymères ou copolymères de chlorure de diméthyldiallylammonium et d'acrylamide, vendus sous les dénominations « MERQUAT 100 », « MERQUAT 550 » et « MERQUAT S » par la Société CALGON, les polysaccharides cationiques tels que les gommes de guar modifiées par du chlorure de 2,3-époxypropyl triméthylammonium commercialisées par exemple sous la dénomination « JAGUAR C13S » par la société MEYHALL et les copolymère vinylpyrrolidone / méthacrylamidopropyl diméthylamine commercialisés notamment sous la dénomination STYLEZE CC 10 par la société ISP et leurs mélanges.

Selon l'invention, le ou les polymères cationiques peuvent représenter de 0,01 % à 10 % en poids, de préférence de 0,05 % à 5 % en poids, et encore plus préférentiellement de 0,1 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Les additifs sont généralement présents dans les compositions selon l'invention en une proportion comprise entre 0,01 % et 20 % en poids, et de préférence entre 0,02 % et 10 % en poids par rapport au poids total de la composition.

Les compositions selon l'invention sont utilisées de préférence comme shampooings pour le lavage et le traitement cosmétique des cheveux et du cuir chevelu et ils sont appliqués, dans ce cas-là, sur des cheveux humides ou secs dans des quantités efficaces pour les laver, cette application étant suivie d'un rinçage de préférence à l'eau, après un éventuel temps de pose.

La présente invention a également pour objet un procédé de traitement cosmétique comprenant l'application sur les cheveux ou le cuir chevelu d'une composition telle que définie précédemment cette application étant suivie d'un rinçage de préférence à l'eau, après un éventuel temps de pose.

Les exemples qui suivent illustrent la présente invention.

### Détermination de l'activité fongistatique

L'activité fongistatique des compositions A à D a été déterminée par une méthode classique dite de CMI (Concentration Minimale Inhibitrice), la CMI correspondant à la concentration la plus faible à laquelle un produit donné inhibe la croissance d'une souche donnée dans des conditions définies.

En l'occurence, des souches de Malassezia Ovalis (ou Pityrospotum ovale) CIP 1363.82 ont été utilisées.

La détermination de l'activité fongistatique des produits a donc été réalisée par la méthode des dilutions en milieu gélosé

100 µl de produit ont été ajoutés à 1.9 ml de milieu de culture gélosé en surfusion à 45°C.

Des dilutions successives, en progression géométrique de raison 2, de la suspension obtenue ont tété réalisées à l'aide du milieu de culture gélosé en surfusion dans les puits d'une plaque (Falcon, 24 puits). Après solidification du milieu, 4µl de la suspension microbienne ont été déposés en surface à l'aide d'une micropipette. Après 48H d'incubation à 30°C, la concentration minimale inhibitrice (C.M.I.) était donnée par la plus faible concentration de produit qui inhibait la croissance microbienne.

La concentration de l'agent antimicrobien à partir de laquelle la croissance du micro-organisme est complètement inhibée (absence de trouble du milieu) est ainsi déterminée et exprimée en dilution.

Les résultats sont rassemblés dans le tableau suivant :

| | B Invention | C | D |
|---|---|---|---|
| Pyrithione de Zn | 0,96 gMA | 0,96 gMA | - |
| Acide citrique | 3 g | 3 g | - |
| Cocoylbétaïne (DEHYTHON AB 30) | - | - | 1,2 g MA |
| APG (KAG 40 de KAO) | 13,8gMA | - | |
| Lauryléther sulfate de sodium (2,2 OE) | - | 13,8 gMA | 12,6 gMA |
| Sulfosuccinate de Na | - | - | |
| Eau qs | 100 g | 100 g | 100 g |
| **CMI** | **1/2560** | 1/1280 | 1/80 |

Plus la CMI est faible, plus la composition est efficace.

## Revendications

1. Composition cosmétique de lavage et de traitement antipelliculaire des cheveux et du cuir chevelu, **caractérisée par le fait qu'**elle contient dans un milieu aqueux :
a) au moins un agent antipelliculaire choisi parmi les sels de pyridinethione
b) au moins un agent tensioactif choisi parmi les tensioactifs non-ionique du type alkyl(poly)glycoside;
c) au moins 1% en poids d'au moins un hydroxyacide.

2. Composition selon la revendication 1, **caractérisée par le fait que** les sels de pyridinethione sont choisis parmi les sels de calcium, de magnésium, de barium, de strontium, de zinc, de cadmium, d'étain et de zirconium.

3. Composition selon la revendication 1 ou 2 , **caractérisée par le fait que** le sel de pyridinethione est le sel de zinc.

4. Composition selon l'une quelconque des revendications 1 à 3, **caractérisée par le fait que** ledit agent antipelliculaire est présent dans des proportions allant de 0,1 à 5% en poids par rapport au poids total de la composition.

5. Composition selon l'une quelconque des revendications précédentes, **caractérisée par le fait que** l'hydroxyacide est présent sous forme d'acide libre et/ou sous la forme de l'un de ses sels associés et/ou sous la forme du lactide correspondant.

6. Composition selon l'une quelconque des revendications précédentes, **caractérisée par le fait que** l'hydroxyacide est choisi parmi les acides citrique, lactique, méthyllactique, phényllactique, malique, mandélique, glycolique, tartronique, tartrique, gluconique, benzylique et l'acide 2-hydroxycaprylique.

7. Composition selon l'une quelconque des revendications 1 à 6, **caractérisée par le fait que** ledit hydroxy acide est présent dans des proportions allant de 2 à 10% en poids par rapport au poids total de la composition.

8. Composition selon l'une quelconque des revendications 1 à 7, **caractérisée par le fait que** ledit agent tensioactif non ionique de type alkyl(poly)glycoside est un composé de formule (I):
R₁-O-(R₂O)ₜ-(G)ᵥ (I)
dans laquelle R, représente un radical alkyle linéaire ou ramifié, saturé ou insaturé, comportant de 8 à 24 atomes de carbone, un radical alkylphényle dont le radical alkyle linéaire ou ramifié comporte de 8 à 24 atomes de carbone, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.

9. Composition selon la revendication 8, **caractérisée par le fait que** dans la formule (I), R₁ désigne un radical alkyle saturé ou insaturé, linéaire ou ramifié comportant de 8 à 14 atomes de carbone, t prend la valeur 0, G désigne le glucose, v prend une valeur de 1 à 4.

10. Composition selon l'une quelconque des revendications 1 à 9, **caractérisée par le fait que** le(s) agent(s) tensioactif(s) non ioniques de type alkyl(poly)glycoside sont présents à raison de 0,5 à 15% en poids par rapport au poids total de la composition.

11. Composition selon l'une quelconque des revendications 1 à 10, **caractérisée par le fait qu'**elle comprend en outre au moins un agent tensioactif anionique.

12. Composition selon la revendication 11, **caractérisée par le fait que** ledit tensioactif est présent en une quantité comprise entre 0,1% et 40% en poids environ, de préférence entre 3% et 30% par rapport au poids total de la composition.

13. Composition selon l'une quelconque des revendications précédente, **caractérisée par le fait qu'**elle comprend en outre un additif choisi parmi les agents tensioactifs cationiques, les polymères anioniques ou non ioniques ou amphotères ou cationiques, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les silicones, volatiles ou non volatiles, solubles et insolubles dans le milieu, les huiles végétales, les huiles de synthèses telles que les poly-alphaoléfines, les huiles fluorées ou perfluorées, et leurs mélanges.

14. Composition selon la revendication précédente, **caractérisée par le fait que** ledit polymère cationique est choisi parmi les dérivés d'éther de cellulose quaternaires, les cyclopolymères, les polysaccharides cationiques, les copolymères vinylpyrrolidone /méthacrylamidopropyl dimethylamine et leurs mélanges.

15. Compositions selon l'une quelconque des revendications 13 ou 14, **caractérisées par le fait que** ledit polymère cationique est présent à une teneur pondérale comprise entre 0,01 % et 10 % par rapport au poids total de la composition et de préférence comprise entre 0,05 % et 5 %.

16. Compositions selon l'une quelconque des revendications précédentes, **caractérisées par le fait qu'**elles présentent un pH compris entre 4 et 9.

17. Utilisation cosmétique d'une composition telle définie à l'une quelconque des revendications précédentes pour le lavage et le traitement antipelliculaire des cheveux et du cuir chevelu.

18. Procédé de traitement de traitement cosmétique comprenant l'application sur les cheveux ou le cuir chevelu d'une composition telle que définie précédemment cette application étant suivie d'un rinçage de préférence à l'eau, après un éventuel temps de pose.

## Claims

1. Cosmetic composition for the washing and the antidandruff treatment of the hair and scalp, **characterized in that** it comprises, in an aqueous medium:
a) at least one antidandruff agent chosen from pyridinethione salts;
b) at least one surface-active agent chosen from nonionic surfactants of the alkyl(poly)glycoside type;
c) at least 1% by weight of at least one hydroxy acid.

2. Compostion according to Claim 1, **characterized in that** the pyridinethione salts are chosen from calcium, magnesium, barium, strontium, zinc, cadmium, tin and zirconium salts.

3. Composition according to Claim 1 or 2, **characterized in that** the pyridinethione salt is the zinc salt.

4. Composition according to any one of Claims 1 to 3, **characterized in that** the said antidandruff agent is present in proportions ranging from 0.1 to 5% by weight, with respect to the total weight of the composition.

5. Composition according to any one of the preceding claims, **characterized in that** the hydroxy acid is present in the form of the free acid and/or in the form of one of its associative salts and/or in the form of the corresponding lactide.

6. Composition according to any one of the preceding claims, **characterized in that** the hydroxy acid is chosen from citric, lactic, methyllactic, phenyllactic, malic, mandelic, glycolic, tartronic, tartaric, gluconic, benzylic and 2-hydroxycaprylic acids.

7. Composition according to any one of Claims 1 to 6, **characterized in that** the said hydroxy acid is present in proportions ranging from 2 to 10% by weight, with respect to the total weight of the composition.

8. Composition according to any one of Claims 1 to 7, **characterized in that** the said nonionic surface-active agent of alkyl(poly)glycoside type is a compound of formula (I):
R₁-O-(R₂O)ₜ-(G)ᵥ (I)
in which R₁ represents a saturated or unsaturated, linear or branched alkyl radical comprising from 8 to 24 carbon atoms or an alkylphenyl radical in which the linear or branched alkyl radical comprises from 8 to 24 carbon atoms, R₂ represents an alkylene radical comprising from 2 to 4 carbon atoms, G represents a reduced sugar comprising from 5 to 6 carbon atoms, t denotes a value ranging from 0 to 10 and v denotes a value ranging from 1 to 15.

9. Composition according to Claim 8, **characterized in that**, in the formula (I), R₁ denotes a saturated or unsaturated, linear or branched alkyl radical comprising from 8 to 14 carbon atoms, t takes the value 0, G denotes glucose and v takes a value from 1 to 4.

10. Composition according to any one of Claims 1 to 9, **characterized in that** the nonionic surface-active agent(s) of alkyl(poly)glycoside type are present in a proportion of 0.5 to 15% by weight, with respect to the total weight of the composition.

11. Composition according to any one of Claims 1 to 10, **characterized in that** it additionally comprises at least one anionic surface-active agent.

12. Composition according to Claim 11, **characterized in that** the said surface-active agent is present in an amount of between 0.1% and 40% by weight approximately, preferably between 3% and 30%, with respect to the total weight of the composition.

13. Composition according to any one of the preceding claims, **characterized in that** it additionally comprises an additive chosen from cationic surface-active agents, anionic or nonionic or amphoteric or cationic polymers, proteins, protein hydrolysates, natural or synthetic waxes, ceramides, pseudoceramides, fatty acids comprising linear or branched C₁₆-C₄₀ chains, such as 18-methyleicosanoic acid, carboxylic acid fatty esters, fatty acid mono-, di- or triglycerides, vitamins, provitamins, such as panthenol, volatile or nonvolatile silicones which are soluble and insoluble in the medium, vegetable oils, synthetic oils, such as poly(α-olefins), fluorinated or perfluorinated oils, and their mixtures.

14. Composition according to the preceding claim, **characterized in that** the said cationic polymer is chosen from quaternary cellulose ether derivatives, cyclopolymers, cationic polysaccharides, vinylpyrrolidone/methacrylamidopropyldimethylamine copolymers and their mixtures.

15. Compositions according to either one of Claims 13 and 14, **characterized in that** the said cationic polymer is present at a content by weight of between 0.01% and 10% with respect to the total weight of the composition and preferably of between 0.05% and 5%.

16. Compositions according to any one of the preceding claims, **characterized in that** they exhibit a pH of between 4 and 9.

17. Cosmetic use of a composition as defined in any one of the preceding claims for the washing and the antidandruff treatment of the hair and scalp.

18. Cosmetic treatment process comprising the application to the hair or to the scalp of a composition as defined above, this application being followed by rinsing, preferably with water, after an optional period of standing.

## Patentansprüche

1. Kosmetische Zusammensetzung zum Waschen und zur Behandlung der Haare und der Kopfhaut gegen Schuppen, **dadurch gekennzeichnet, dass** sie in einem wässerigen Medium enthält:
a) mindestens einen Wirkstoff gegen Schuppen, der unter den Pyridinthion-Salzen ausgewählt ist,
b) mindestens einen grenzflächenaktiven Stoff, der unter den nichtionischen grenzflächenaktiven Stoffen vom Alkyl(poly)-glykosid-Typ ausgewählt ist, und
c) mindestens 1 Gew.-% mindestens einer Hydroxysäure.

2. Zusammensetzung nach Anspruch 1, **dadurch gekennzeichnet, dass** die Pyridinthion-Salze unter den Calcium-, Magnesium-, Barium-, Strontium-, Zink-, Cadmium-, Zinn- und Zirkonium-Salzen ausgewählt sind.

3. Zusammensetzung nach Anspruch 1 oder 2, **dadurch gekennzeichnet, dass** es sich bei dem Pyridinthion-Salz um das Zinksalz handelt.

4. Zusammensetzung nach einem der Ansprüche 1 bis 3, **dadurch gekennzeichnet, dass** der Wirkstoff gegen Schuppen in Mengenanteilen von 0,1 bis 5 Gew.-%, bezogen auf das Gesamtgewicht der Zusammensetzung, vorliegt.

5. Zusammensetzung nach einem der vorhergehenden Ansprüche, **dadurch gekennzeichnet, dass** die Hydroxysäure als freie Säure und/oder in der Form eines ihrer zugehörigen Salze und/oder in der Form des entsprechenden Lactids vorliegt.

6. Zusammensetzung nach einem der vorhergehenden Ansprüche, **dadurch gekennzeichnet, dass** die Hydroxysäure unter Citronensäure, Milchsäure, Methylmilchsäure, Phenylmilchsäure, Äpfelsäure, Mandelsäure, Glykolsäure, Tartronsäure, Weinsäure, Gluconsäure, 2,2-Diphenyl-2-hydroxyethansäure und 2-Hydroxycaprylsäure ausgewählt ist.

7. Zusammensetzung nach einem der Ansprüche 1 bis 6, **dadurch gekennzeichnet, dass** die Hydroxysäure in Mengenanteilen von 2 bis 10 Gew.-%, bezogen auf das Gesamtgewicht der Zusammensetzung, vorliegt.

8. Zusammensetzung nach einem der Ansprüche 1 bis 7, **dadurch gekennzeichnet, dass** es sich bei dem nichtionischen grenzflächenaktiven Stoff vom Alkyl(poly)glykosid-Typ um eine Verbindung der Formel (I) handelt:
R₁-O-(R₂O)ₜ-(G)ᵥ (I),
worin bedeuten:
- R₁ eine geradkettige oder verzweigte, gesättigte oder ungesättigte Alkylgruppe mit 8 bis 24 Kohlenstoffatomen oder eine Alkylphenylgruppe, deren geradkettige oder verzweigte Alkylgruppe 8 bis 24 Kohlenstoffatome aufweist,
- R₂ eine Alkylengruppe mit 2 bis 4 Kohlenstoffatomen,
- G einen reduzierten Zucker, der 5 bis 6 Kohlestoffatome aufweist,
- t einen Wert im Bereich von 0 bis 10 und
- v einen Wert im Bereich von 1 bis 15.

9. Zusammensetzung nach Anspruch 8, **dadurch gekennzeichnet, dass** in der Formel (I) R₁ eine gesättigte oder ungesättigte, geradkettige oder verzweigte Alkylgruppe mit 8 bis 14 Kohlenstoffatomen, t den Wert 0 und G Glucose bedeuten und v einen Wert von 1 bis 4 einnimmt.

10. Zusammensetzung nach einem der Ansprüche 1 bis 9, **dadurch gekennzeichnet, dass** der (die) nichtionische(n) grenzflächenaktive(n) Stoff(e) vom Alkyl(poly)glykosid-Typ in einem Mengenanteil von 0,5 bis 15 Gew.-%, bezogen auf das Gesamtgewicht der Zusammensetzung, vorliegen.

11. Zusammensetzung nach einem der Ansprüche 1 bis 10, **dadurch gekennzeichnet, dass** sie ferner mindestens einen anionischen grenzflächenaktiven Stoff enthält.

12. Zusammensetzung nach Anspruch 11, **dadurch gekennzeichnet, dass** der grenzflächenaktive Stoff in einer Menge von etwa 0,1 bis 40 Gew.-% und vorzugsweise von 3 bis 30 Gew.-%, bezogen auf das Gesamtgewicht der Zusammensetzung, vorliegt.

13. Zusammensetzung nach einem der vorhergehenden Ansprüche, **dadurch gekennzeichnet, dass** sie außerdem einen Zusatzstoff enthält, der unter den kationischen grenzflächenaktiven Stoffen, anionischen, nichtionischen, amphoteren oder kationischen Polymeren, Proteinen, Proteinhydrolysaten, natürlichen oder synthetischen Wachsen, Ceramiden, Pseudoceramiden, Fettsäuren mit geraden oder verzweigten C₁₆₋₄₀-Ketten, beispielsweise 18-Methyleicosansäure, Fettestern von Carbonsäuren, Mono-, Dioder Triglyceriden von Fettsäuren, Vitaminen, Provitaminen, wie Panthenol, flüchtigen oder nicht flüchtigen Siliconen, die in dem Medium löslich oder unlöslich sind, pflanzlichen Ölen, synthetischen Ölen, wie Poly-α-olefinen, fluorierten oder perfluorierten Ölen und deren Gemischen ausgewählt ist.

14. Zusammensetzung nach dem vorhergehenden Anspruch, **dadurch gekennzeichnet, dass** das kationische Polymer unter den quartären Celluloseetherderivaten, den Cyclopolymeren, den kationischen Polysacchariden, den Copolymeren von Vinylpyrrolidon und Methacrylamidopropyldimethylamin sowie deren Gemischen ausgewählt ist.

15. Zusammensetzungen nach einem der Ansprüche 13 oder 14, **dadurch gekennzeichnet, dass** das kationische Polymer in einem Gewichtsanteil von 0,01 bis 10 % und vorzugsweise von 0,05 bis 5 %, bezogen auf das Gesamtgewicht der Zusammensetzung, vorliegt.

16. Zusammensetzungen nach einem der vorhergehenden Ansprüche, **dadurch gekennzeichnet, dass** sie einen pH-Wert von 4 bis 9 aufweisen.

17. Kosmetische Verwendung einer wie in einem der vorhergehenden Ansprüche definierten Zusammensetzung zum Waschen und zur Behandlung der Haare und der Kopfhaut gegen Schuppen.

18. Verfahren zur kosmetischen Behandlung, das die Applikation einer wie vorstehend definierten Zusammensetzung auf das Haar oder die Kopfhaut umfasst, wobei im Anschluss an die Applikation gegebenenfalls nach einer Einwirkzeit vorzugsweise mit Wasser gespült wird.
